# EUROPÄISCHE PATENTSCHRIFT

(11) **EP 0 768 838 B1**
(45) Veröffentlichungstag und Bekanntmachung des Hinweises auf die Patenterteilung: **05.08.1998**
(21) Anmeldenummer: 95922454.4
(22) Anmeldetag: 16.05.1995
(51) Int. Cl.: A61B 17/04

(54) **CHIRURGISCHES NÄHGERÄT**
SURGICAL SUTURING DEVICE
INSTRUMENT CHIRURGICAL DE SUTURE

(30) Priorität: 07.07.1994 DE 4423881
(43) Veröffentlichungstag der Anmeldung: 23.04.1997
(73) Patentinhaber: Forschungszentrum Karlsruhe GmbH, 76133 Karlsruhe (DE)
(72) Erfinder: WURSTER, Helmut, D-75038 Oberderdingen (DE); TRAPP, Rainer, D-76676 Graben-Neudorf (DE)
(74) Vertreter: Rückert, Friedrich, Dr.
(86) Internationale Anmeldenummer: EP9501849
(87) Internationale Veröffentlichungsnummer: WO9601587

(56) Entgegenhaltungen:
- EP-A- 0 535 906
- DE-A- 2 447 719
- DE-C- 4 124 383
- DE-C- 4 139 628

## Beschreibung

Die Erfindung betrifft ein chirurgisches Nähgerät, insbesondere für die endoskopische Chirurgie, nach dem Oberbegriff des Anspruchs 1.

Mit einem solchen Nähgerät werden im Körper des Patienten unter endoskopischer Beobachtung, also unter minimaler Öffnung des Leibes oder durch natürliche Körperöffnungen hindurch, chirurgische Nähte gelegt.

Aus DE 41 24 381, DE 41 24 383 und DE 41 39 628 sind zwei Nähgeräte und eine Betätigungseinrichtung bekannt. Bei den beiden Nähgeräten handelt es sich um ein Gerätprinzip, nämlich ein Nähkopf mit einem feststehenden und einem beweglichen Maulteil, der über einen Schaft von einem externen Handgriff und einem Pedalschalter betätigt wird. Für den Nähvorgang wird zwischen den Maulteilen eine Nadel mit einer Öse in der Mitte umgesetzt. Die Nadel ist immer in einem der Maulteile kraft- und formschlüssig eingespannt. Es sind zwei getrennte Betätigungsvorgänge notwendig, um den Nähvorgang zu führen. Einmal für die Bewegung des beweglichen Maulteils, das andere Mal für das Arretieren oder Freigeben der Nadel in einem der Maulteile.

Ein weiteres chirurgisches Nähgerät für die endoskopische Chirurgie ist aus der Offenlegungsschrift DE 42 01 337 bekannt. Bei diesem darin beschriebenen Instrument wird eine bogenförmige Nadel durch die Kraft eines während der Bewegung von gebogenen Rohrstücken, in denen die Nadel angeordnet ist, zu- und abschaltbaren Magneten gehalten.

Für den Chirurgen ist es stets eine Erleichterung und damit ein Vorteil, wenn die Handgriffe an den Instrumenten auf ein notwendiges Minimum beschränkt bleiben und Verbindungseinrichtungen von den Operationsinstrumenten zu peripheren Geräten vermieden werden können.

Von daher stellte sich die Aufgabe zur Entwicklung eines chirurgischen Nähgeräts, hauptsächlich für die endoskopische Chirurgie, bei dem der Nähvorgang und das Umsetzen der Nadel zwischen den beiden Maulteilen mit einem Handgriff durchgeführt werden kann.

Diese Aufgabe führte zu der Erfindung des gattungsgemäßen Nähgeräts mit dem Antriebs- und Verriegelungsmechanismus und dem Nähkopf mit seinen Maulteilen und der Nadelführung darin, samt der Nähnadel dafür, wie es im Anspruch 1 durch die kennzeichnenden Merkmale erläutert ist.

In den Unteransprüchen sind Ausgestaltungen gekennzeichnet, die eine Erleichterung für die Fertigung und die Bedienung des chirurgischen Nähinstuments mit sich bringen.

Ein mechanischer Teiler der die Bewegungszahl des Handgriffes im Verhältnis 2:1 herunterteilt wird dazu benutzt um die Klemmvorrichtung an der feststehenden Zangenbranche zu betätigen, so daß durch jede zweite Bewegung des Handgriffes diese Klemmvorrichtung betätigt und die Nadel festgehalten wird. Dadurch wird der Chirurg von der Umschaltung entlastet und braucht sich nicht mehr darum zu kümmern, wann er den Handhebel oder Fußschalter zum Festhalten der Nadel betätigen muß.

Beim fortlaufenden Betätigen des Handgriffs wird die Nadel automatisch von der einen zur anderen Zangenbranche übergeben, und ein fortlaufendes Nähen kann dadurch stattfinden. Diese Erfindung ist sowohl für das axiale als auch für das radiale endoskopische Nähgerät einsetzbar.

Die Arretierung der Nadel wird nicht durch Klemmbacken realisiert sondern durch einen im Maulteil beweglichen, an seiner Stirnseite vorn abgerundeten Stift, welcher in eine Kerbe der Nadel eingreift und so diese in ihrer Führungshülse arretiert.

Um beim radialen Nähgerät, welches eine gebogene oder eine in der Mitte geknickte Nadel benötigt, muß diese gegen Verdrehen gesichert werden. Es zeigt sich dabei, daß eine dreikantige Nadelform diese Forderung voll erfüllt und einen einwandfreien Sitz der Nadel garantiert. Die dreikantige Nadel läuft spitz aus, wobei die untere Kante leicht eben angeschrägt ist, um ein besseres Einführen in das entsprechend geformte Nadelführungsteil zu gewährleisten.

Eine weitere Lösung der Aufgabe besteht darin, daß die durch den Stift an der Kerbe gehaltene Nadel, nicht wie in den Schriften DE 41 24 381 und DE 41 24 383 beschrieben, auf der einen Seite fest arretiert wird und auf der Gegenseite beim beweglichen Maulteil über Federkraft mit einer bestimmten Abzugskraft gehalten wird, sondern daß beide Maulteile über einen beweglichen durch den Umschaltmechanismus betätigten Stift verfügen, welcher dann welchselseitig die Nadel fest in dem jeweilig angesteuterten Maulteil arretiert. Durch diese Maßnahme wird erreicht, daß die Nadel in jedem Maulteil absolut fest sitzt und nach der erfolgten Übernahme durch z.B. starkes Ziehen am Faden nicht mehr verloren gehen kann. Andererseits wird beim Einführen der Nadel in die Nadelführung die Nadelspitze in keiner Weise mechanisch belastet, weil der die Nadel fixierende halbrunde Stift erst nach dem Einführen der Nadel in die prismatische Nadelhalterung vorgeschoben wird. Dies wird dadurch erreicht, daß nach dem Schließen des Maulteils, das heißt nach dem Einführen der Nadel in das gegenseitige Lager, der Handgriff weiter zusammengedrückt und dadurch erst der Umschaltmechanismus betätigt wird, wobei die bestehende Fixierung gelöst und in dem soeben in die Nadel eingefahrenen Maulteil betätigt wird. Beim nächsten Betätigen des Handgriffes erfolgt die Lösung und Fixierung im jeweils anderen Maulteil, so daß ein einwandfreies Übergeben der Nadel vom einen zum anderen Maulteil uns sicheres Fixieren derselben erfolgt.

Durch diese Maßnahme erhält das chirurgische Nähgerät Vorteile die für seinen praktischen Einsatz erforderliche Einfachheit und Betriebssicherheit gewährleisten.

Durch das Weiterdrücken des Handgriffes nach dem Schließen der Maulteile gegen eine Federkraft, welche die Maulteile geschlossen hält, wird gleichzeitig erreicht, daß auf die Maulteile immer nur der durch die Feder eingestellte maximale Druck zur Wirkung kommt, wodurch eine Überbelastung der Maulteile durch zu starkes Drücken beim Schließen am Handgriff vermieden wird. Dies ist ein zusätzlicher Vorteil.

Das Betätigen des Fixierungsmechanismus im beweglichen Maulteil kann z. B. über einen Stift aus superelastischem Material bewerkstelligt werden, welcher den Maulteilwinkel von 45° Öffnung ohne weiteres mitmacht. Bei der konstruktiven Lösung dieser Aufgabe ist zu beachten, daß der Bewegungsweg des Stiftes beim Bewegen des Maulteils nicht verändert wird, um ein sicheres Festhalten der Nadel zu garantieren.

Zwei Ausführungsbeispiele der Erfindung sind in der Zeichnung dargestellt. Es zeigen:
- Figur 1: den Nähkopf mit dem beweglichen und dem feststehenden Maulteil,
- Figur 2: die Nadelführung im feststehenden Maulteil,
- Figur 3a: den Antriebs- und Verriegelungsmechanismus in Seitenansicht,
- Figur 3b: den Antriebs- und Verriegelungsmechanismus in der Draufsicht,
- Figur 4a: den Nähkopf mit zwei beweglichen Maulteilen,
- Figur 4b: den Schnitt A-A bei geöffneten Maulteilen,
- Figur 4c: den Schnitt A-A bei geschlossenen Maulteilen und
- Figur 4d: den Schnitt B-B,
- Figur 5: die dreikantige Nadel.

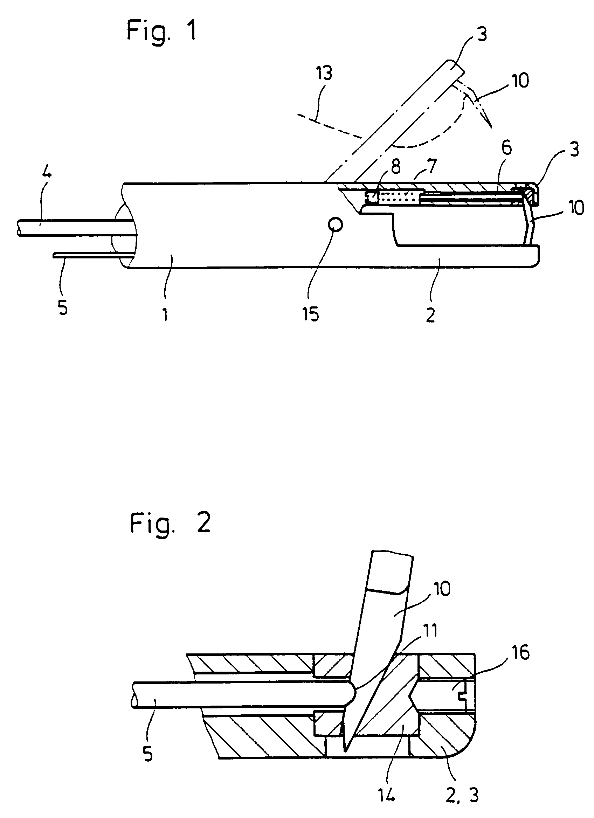

In Figur 1 ist das distale Ende des chirurgischen Nähgeräts mit in Richtung Schaftachse ausgerichtetem Nähkopf und Schaftrohrende 1 dargestellt. Der Nähkopf setzt sich aus dem beweglichen und dem feststehenden Maulteil 3 und 2 zusammen. Das bewegliche Maulteil 3 ist drehbar um die Achse 15 gelagert und wird über die Schubstange 4 auf- und zugeklappt.

Am Ende des beweglichen Maulteils 3 ist die Nadelführung 14 gemäß Figur 2 eingelassen. Im Gegensatz zum feststehenden Maulteil 2 wird die Nadel 10 im beweglichen Maulteil 3 über den Stift 6, der über die Feder 7 vorgespannt wird, fixiert. Die Federkraft ist in Grenzen über die Stellschraube 8 einstellbar. Im feststehenden Maulteil 2 ist die selbe Nadelführung 14 eingelassen. Nur wird hier die Nadel 10 über Schaltstange 5 arretiert, die von der Antriebs- und Verriegelungsmechanik aus betrieben wird.

Die Nadelführung 14 in den Maulteilen 2, 3 wird über die Wurmschraube 16, wie in Figur 2 dargestellt, fixiert. Dort ist auch gezeigt, wie die Schaltstange 5 oder der Stift 6 die Nadelspitze durch die Kerbe 11 formschlüssig in die Nadelführung 14 drückt.

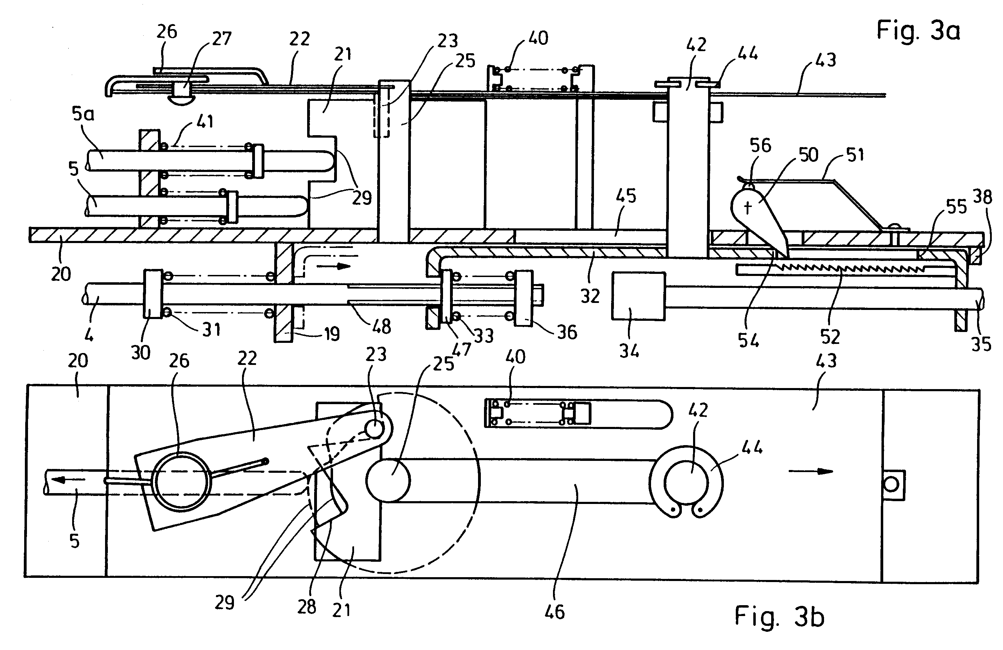

In der Seiten- und Draufsicht zeigen die Figuren 3a und b die Antriebs- und Verriegelungsmechanik, und zwar den Zustand des völlig angezogenen Handgriffs. Die entspannte Situation, gewissermaßen die Ausgangslage, ist durch den in der Figur 3a gestrichelt gezeichneten abgewinkelten Teil des Schiebers 32 am Anschlag 19 der Basisplatte 20 angedeutet. Das Ineinandergreifen der Mechanik von dieser Ausgangslage bis zum völlig angezogen Handgriff und zurück wird im folgenden anhand dieser Figuren beschrieben. Der Handgriff ist hierzu nicht mehr eingezeichnet, da er ein sehr gebräuchliches und geläufiges Bauteil ist. Die Nadel 10 wird in das Maulteil 3 eingesetzt und über den Stift 6 und die Feder 8 gehalten.

Beim Zusammenziehen des Handgriffs bewegt sich der Schieber 32 (nach rechts in Figur 3a) in der eingezeichneten Pfeilrichtung. Der Schieber ist fest mit der Stange 35 verbunden, an der der Handgriff ansetzt. Die Schubstange 4, die durch den Anschlag 19 und den abgewinkelten linken Teil des Schiebers geführt wird, ist über die Feder 33 mit dem Schieber 32 gekoppelt und wird beim Anziehen des Handgriffs ebenfalls nach rechts mit bewegt. Durch diese Bewegung nach rechts klappt das bewegliche Maulteil 3 auf das feststehende zu, und die Nadel 10 fährt in die freie Nadelführung 14 ein.

Diese Bewegung nach rechts macht auch der fest mit dem Schieber 32 verbundene Bolzen 42. Er geht durch das Langloch 45 in der Basisplatte 20 hindurch und hat am freien Ende die Platte 43 auf dem Bund aufliegen, die dort über den Seegerring 44 gesichert ist. Die Platte 43 wird durch die Achse 25 geführt und hat dafür ebenfalls ein Langloch 46 (Figur 3b). Auf der Platte 43 ist auf der Längsachse der Schalthebel 22 durch den Bolzen 27 drehbar gelagert, und zwar so, daß er stets mit seinem freien Ende, an dem der Schaltstift 23 sitzt und durch die Platte 43 greift, über die rücktreibende Kraft der Feder 26 auf die Achslinie zurückschwenkt, falls er frei ist.

Während der bisher geschilderten Bewegung des Handgriffs greift der Umschaltstift 23 in die eine (obere) Hälfte der w-förmigen Ausnehmung 28 des Umschalters 21 ein und dreht diesen in die entsprechende Richtung (nach rechts). Der Umschalter 21 ist drehbar auf der Achse 25 gelagert. Mit ihm sind die beiden gegenläufig geformten Nocken 29 gekoppelt. Sie werden in die entsprechende Drehrichtung mitgenommen und betätigen die Schaltstangen 5 und 5a. Im dargestellten Aufbau bewegen sich die beiden Schaltstangen 5 und 5a dadurch gegenläufig.

Mit einem nicht dargestellten, über Feingewinde wirkenden Einstellmechanismus können die Schaltstangen 5, 5a in ihrer axialen Richtung verschoben werden, so daß die Nadel 10 durch die Nockenstellung gerade fest arretiert ist. Die beiden Federn 41 am Ende der Nocken 29 pressen die die Schaltstangen 5, 5a in jeder Stellung des Umschalters 21 ständig gegen die jeweilige Nocke 29.

Die gezeichnete Situation in den Figuren 3a und b ist so, daß die Schaltstange 5 durch die untere Nocke 29 (Figur 3a) völlig nach vorne gedrückt ist und dadurch die Nadel 10 mit ihrer in der Nadelführung 14 im festen Maulteil 2 eingelegten Spitze formschlüssig fixiert ist.

Eine eingenommene Position auf dem Weg zum Anschlag 38 wird durch den Verriegelungsmechanismus in Form einer Rätsche rechts an der Basisplatte 20 gehalten. Die Rätsche besteht aus der Zahnstange 52 und der Sperrklinke 50 mit der Nocke 56, die über die Feder 51 in Position gehalten wird. Die Zahnstange 52 ist am Schieber 32 befestigt. Die Sperrklinke 50 ist auf der Basisplatte 20 drehbar gelagert und greift mit ihrer Spitze durch einen Durchbruch in der Basisplatte 20 und im Schieber 32 in die Zahnstange 52 ein. So wird ein Rücklaufen des Schiebers 32 zwischen den Anschlägen 19 und 38 verhindert.

Eine neue Position kann nur durch weiteres Durchziehen des Handgriffs erreicht werden. Dies geht so lange, bis der Schieber 32 mit seiner rechten Abwinklung den Anschlag 38 erreicht. Kurz vor Erreichen dieser Position hebt die Durchbruchkante 54 am Schieber 32 die Spitze der Sperrklinke 50 aus der Zahnstange 52. Wird der Handgriff jetzt losgelassen, so geht der Schieber 32 zurück, bis er mit seiner linken Abwinklung am Anschlag 19 auftrifft. Die Feder entspannt sich, bis die verschiebbare Scheibe 47 an ihren Anschlag 48 kommt. Die rechte Durchbruchkante 55 am Schieber 32 stellt die Sperrklinke 50 zurück und läßt die Spitze wieder am Anfang der Zahnstange 52 durch die Wirkung der Plattfeder 51 einrasten. Die Sperrklinke 50 nimmt so durch die Plattfeder 51 zwei stabile Lagen ein. Die Rätsche ist einer von mehreren in Frage kommenden bistabilen Veriegelungsmechanismen.

Auf das bewegliche Maulteil 3 wirkt eine rücktreibende Kraft durch die Feder 31. Da die Nadel 10 im feststehenden Maulteil 2 arretiert ist, kann es dies aufgrund des federnden, eingerasteten Stiftes 6 in der anderen Nadelspitze im freien Maulteil 3 nicht. Um das bewegliche Maulteil 3 zwangsweise vollends aufzuklappen, drückt beim Öffnen des Haltegriffs die Betätigungsstange 35 mit ihrem einstellbaren Ende 34 gegen die Stange 4, so daß die Nadelspitze aus der Nadelführung 14 herausgezogen wird.

Die Nadel 10 sitzt jetzt fest arretiert im feststehenden Maulteil 2, da sich die Lage des Umschalters 21 während des Zurücklaufens des Schiebers nicht verändert hat, wohl aber die Platte 43 zurückfuhr (in der Figur 3a nach links), und zwar durch die rücktreibende Kraft der Feder 40, die zwischen der Platte 43 und der Basisplatte 20 parallel zu dieser gekoppelt ist.

Bei diesem Bewegungsablauf gleitet der Schaltstift 23 am Schalthebel 22 aus der w-förmigen Ausnehmung 28 am Umschalter 21, und der Schalthebel 22 schwinkt durch die rücktreibende Feder 26 auf die Längsachse der Platte 43 zurück. Der Umschalter 21 bleibt zunächst weiter in seiner zuvor eingenommenen Lage und hält die Nadelspitze in der Nadelführung 14 des feststehenden Maulteils 2 arretiert.

Die Nadel wird jetzt durch das zu nähende Gewebe geführt und der Handgriff erneut betätigt, wobei das Maulteil 3 zuklappt und das aufgesammelte Gewebe über die Nadel schiebt. Beim weiteren Durchdrücken des Handgriffs wird der Umschalter 21 über den Schalthebel 22 in die entgegengesetzte Richtung gedreht, weil der Schaltstift jetzt in die andere (untere) Hälfte der w-förmigen Ausnehmung 28 eingreift. Die Arretierung der Nadel 10 im feststehenden Maulteil 2 wird gelöst. Die Nadel 10 ist jetzt im beweglichen Maulteil 3 über den durch Federkraft angedrückten Stift 6 verankert.

Nach völligem Durchdrücken des Handgriffs löst sich die Rätsche und die Maulteile öffnen sich, wie oben geschildert, nur bleibt jetzt die Nadel 10 im beweglichen Maulteil 3. Sie kann jetzt vollends durch das Gewebe gezogen, der Faden angespannt, und ein neuer Stich mit Nadelumsetzen eingeleitet werden.

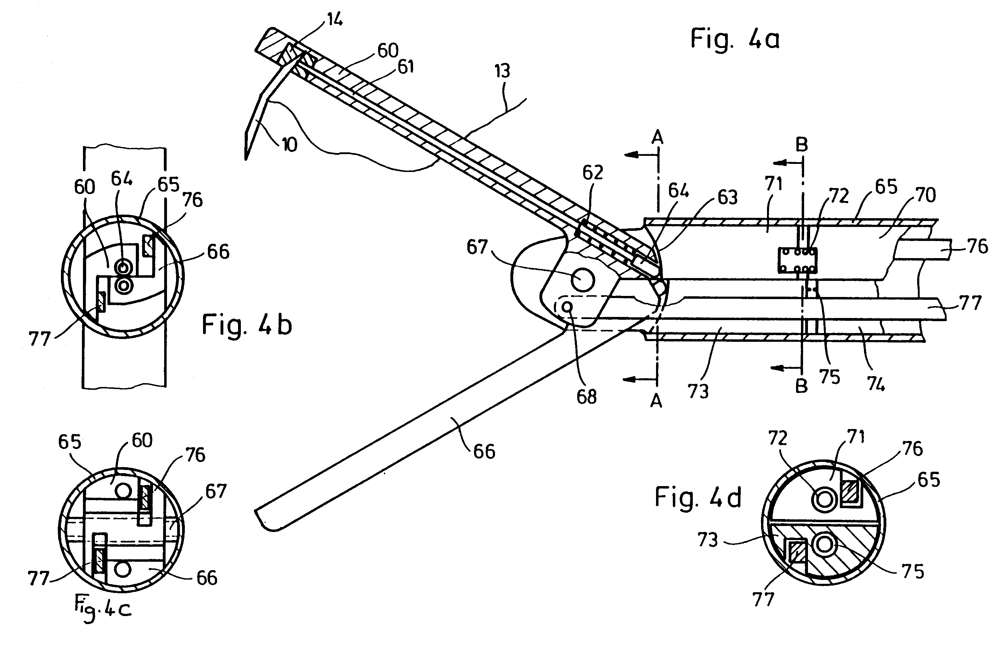

Die Ausgestaltung des chirurgischen Nähgeräts mit zwei beweglichen Maulteilen 60, 66 ist in der Figur 4a dargestellt und wird daran und mit den Schnitten, Figuren 4b, c, d erläutert.

Die Maulteile 60, 66 sind in der Achse 67 des Schaftrohrs 65 gelagert. Beide Maulteile 60, 66 werden über die Stangen 76 und 77 an den Gelenken 68 betätigt. Die Stangen 76, 77 werden weiter hinten zusammengeführt und gemeinsam von der Schubstange 4 getrieben. Die Maulteile 60, 66 sind identisch. Sie haben eine Längsbohrung zur Führung des Arretierungsstifts 61. Die Bohrung ist in Gelenknähe erweitert, um die den Stift 61 rücktreibende Feder 62 aufzunehmen und für die Bewegung des dicken Stiftendes 64 Platz zu haben.

Der Schieber 71 für das in der Figur 4a obere Maulteil 60 ist über die Feder 72 mit dem Teil 70 verbunden. Er treibt den Arretierungsstift 61 an seinem dicken Stiftende 64 an. Entsprechendes gilt für das untere Maulteil 66, das von der Stange 77 und dessen Arretierungsstift 61 von dem Schieber 73 getrieben wird. Auch dort ist das Teil 74 über die Feder 75 mit den Schieber 73 verbunden. Diese beiden Antriebsteile für die Nadelarretierung liegen symmetrisch zur Schaftachse. Das ist auch aus den Schnitten A-A, Figur 4b, c und B-B, Figur 4d zu entnehmen.

Die Schieber 71 und 73 haben die den dicken Stiftenden 64 zugewandte Stirnflächen 63, die so geformt sind, daß die Lage des jeweiligen Stiftes zum Maulteil 60 und 66 in jeder Lage bestehen bleibt. Dort treiben die Schieber 71 und 73 den zugehörigen Arretierungsstift 61 an seinem dicken Stiftende 64. Beide Teile 70 und 74 sind mit den Schaltstangen 5 und 5a gekoppelt und werden, wie oben erläutert und aus den Figuren 3a und b ersichtlich, durch die Nocken 29 gegenläufig getrieben. Dadurch geht aufgrund der oben geschilderten Handgriffbetätigung die Nadel 10 abwechselnd in die Nadelführung 14 der Maulteile 60 und 66 über und arretiert.

An dieser Stelle sei darauf hingewiesen, daß dieser Arretierungsmechanismus mit den beiden Schaltstangen 5 und 5a ohne Probleme auch für den Nähkopf mit beweglichem und festem Maulteil 3 und 2 verwendet werden kann, so daß dann die Federarretierung im beweglichen Maulteil 3, wie oben geschildert, entfiele und der schaltstangenantrieb dafür eingebaut wäre.

Beim Loslassen des Handgriffs bleiben die Nocken 29 stehen. Das dicke Ende der Stifte 64 gleitet, wenn die Maulteile 60 und 66 geöffnet werden, auf der jeweiligen Stirnfläche 63 der Schieber 71 und 73, die so geformt sind, daß die Lage der Stifte 61 zum jeweiligen Maulteil 60 und 66 bestehen bleibt. Die Nadel 10 bleibt dadurch in der gerade eingelegten Nadelhalterung 14 arretiert, sie kann nicht herausfallen. Diese Art der Arretierung ist sehr zuverlässig und kann stark belastet werden.

Wird der durchgezogene und wieder freigegebene Handgriff erneut zusammengezogen, so wird nach dem Aufnehmen der Nadel 10 in beiden Nadelführungen 14 das weitere Zusammenklappen der beiden Maulteile 60 und 66 durch die Nadel 10 blockiert (siehe Beschreibung oben). Beim weiteren Zusammenziehen des Handgriffs wird der Umschalter 21 über den jetzt in der anderen (unteren) Hälfte der w-förmigen Ausnehmung 28 eingreifenden Schaltstift 23 am Schalthebel 22 in die andere (linke) Drehrichtung gefahren. Die beiden Schaltstanngen 5 und 5a laufen gegenläufig durch die Formen der beiden Nocken 29. Dadurch wird die arretierte Nadelspitze freigegeben und die andere arretiert.

Werden die Maulteile 60 und 66 erneut aufgeklappt, so kann die Nadel 10 mit dem durch Öse 13 gefädelten Faden vollends durch das Gewebe gezogen werden und ein neuer Stich begonnen werden.

Figur 4b zeigt den in Figur 4a eingezeichneten Schnitt A-A am Beginn des Nähkopfs, wenn beide Maulteile 60 und 66 wie in Figur 4a geöffnet sind. Sichtbar sind die beiden Anfänge der Maulteile 60 und 66 sowie die dickeren, abgerundeten Stiftenden 64 der beiden Arretierungsstifte 61. Figur 4c zeigt den selben Schnitt für die geschlossenen Maulteile 60, 66. Figur 4d schließlich zeigt den Schnitt B-B durch das Ende des Schafts 65, im Bereich der Federn 72 an der Stoßstelle zwischen dem Teil 70 und 17 sowie zwischen Teil 73 und 74.

Die Handhabung des Instrument ist einfach und die Nadelsicherung ist sehr zuverlässig. Wie oben erwähnt, kann die Nadel 10 auch nur durch Federkraft arretiert werden, allerdings birgt das die Gefahr in sich, daß bei zähem Gewebe die Nadel 10 aufgrund ihrer federnden Arretierung und der verhältnismäßig großen Kräfte an ihr beim Durchziehen durch das Gewebe aus der Nadelführung herausgezogen wird. Das kann bei dem Arretierungsmechanismus, wie in Figur 4a, b, c und d dargestellt nicht so ohne weiteres eintreten.

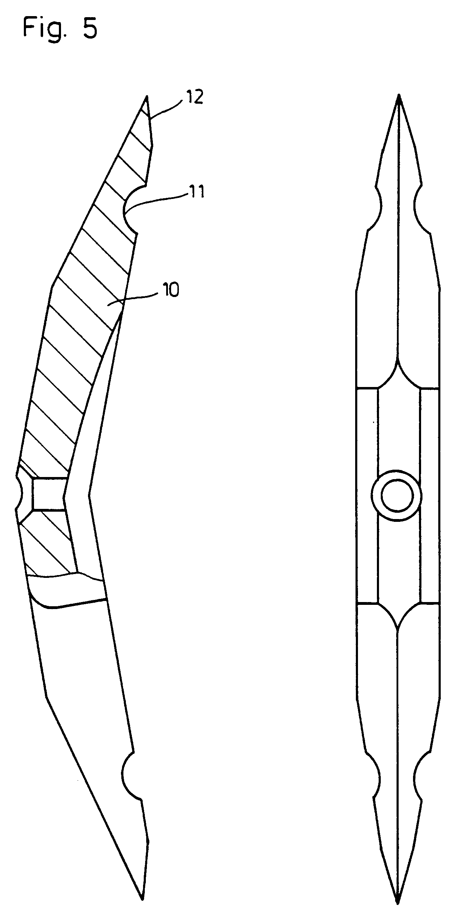

Der dreieckige Nadelquerschnitt nach Figur 5a und b unterstützt die Arretierung. Die Krümmung der Nadel 10, die in etwa der Kreisbahn der Nadelführung 14 entspricht, ermöglich ein reißfreies Durchstechen von Gewebe. Die Kerbe an der Nadel 10 ist zylinderförmig. Da sich die Nadelspitze formschlüssig in die Nadelführung einlegt, ist ein Verkippen der Nadel 10 nach dem Andrücken durch den Arretierungsstift 6 oder 61 nicht mehr möglich.

Die in den Figuren 5a, b dargestellte Form mit dem gratförmigen Rücken der Nadel 10 kann auch der Gestalt sein, daß der Rücken eine Breitseite hat und innen von Nadelspitze zu Nadelspitze die Gratlinie verläuft. Das ist unter Umständen für die zylindrischen Kerben 11 zweckmäßig, da sie dann mehr im vollen Nadelmaterial liegen und die Nadelspitzen stabiler bleiben.

Der Antriebs- und Arretierungsmechanismus ist nicht auf das krokodilförmige Maulteil beschränkt. Nähköpfe, die senkrecht oder schräg von der Schaftachs abstehen, sind über die beschriebenen Mechanismen ebenfalls anzutreiben. Hierbei sind allenfalls bekannte konstruktive Modifikationen beim Übergang vom Schaftende zum Nähkopf zu berücksichtigen.

### Bezugszeichenliste

- 1: Schaftrohr
- 2: feststehendes Maulteil
- 3: bewegliches Maulteil
- 4: Schubstange
- 5: Schaltstange
- 5a: Schaltstange
- 6: Stift
- 7: Feder
- 8: Schraube, Stellmutter
- 10: Nadel
- 11: Kerbe
- 13: Öse
- 14: Nadelführung
- 15: Achse
- 16: Fixierschraube
- 19: Anschlag
- 20: Basisplatte
- 21: Umschalter
- 22: Schalthebel
- 23: Schaltstift
- 25: Achse
- 26: Feder
- 27: Bolzen
- 28: w-förmige Ausnehmung
- 29: Nocke
- 31: Feder
- 32: Schieber
- 33: Feder
- 34: Ende
- 35: Stange
- 38: Anschlag
- 40: Feder
- 41: Feder
- 42: Bolzen
- 43: Platte
- 44: Seegerring
- 45: Langloch
- 46: Langloch
- 47: Scheibe
- 50: Sperre
- 51: Feder
- 52: Zahnstange
- 55: Kante
- 56: Nocke
- 60: Maulteil
- 61: Arretierungsstift
- 62: Feder
- 63: Stirnfläche
- 64: Stiftende
- 65: Schaftrohr
- 66: Maulteil
- 67: Achse
- 70: Teil
- 71: Schieber
- 72: Feder
- 73: Schieber
- 74: Teil
- 75: Feder
- 76: Stange

## Patentansprüche

1. Chirurgisches Nähgerät, insbesondere für die endoskopische Chirurgie, bestehend aus:
a) einem Handgriff mit feststehendem und beweglichen Teil und einer darauf aufgesetzten Antriebs- und Verriegelungsmechanik,
b) einem an der Antriebs- und Verriegelungsmechanik ansetzenden Schaft, in dem Koppeleinrichtungen zu einem aufgesetzten, zangenförmigen Nähkopf verlaufen, wobei der in der Verlängerung der Schaftachse liegenden Nähkopf aus zwei, über eine der Koppeleinrichtungen, der Schubstange, aufeinander zu und weg klappbaren Maulteilen besteht, von denen mindestens eines auf die Schaftachse zu und wegdrehbar gelagert ist, und je einer Nadelführung am Ende der beiden Maulteile,
c) einer Nadel mit Mittelöse, die mit ihren Spitzen in die Nadelführung formschlüssig eingespannt und zwischen beiden Nadelführungen über Handgriffbetätigung umgesetzt werden kann,
dadurch gekennzeichnet, daß
die Antriebs- und Verriegelungsmechanik, die auf einer Basisplatte (20) montiert ist:
d) aus einer starr mit einem Schieber (32) gekoppelten Stange (35) zur Bewegung des beweglichen Maulteils (3 oder 60 und 66) besteht, die über den Handgriff entlang der Längsachse der Basisplatte (20) angezogen wird, und der Schieber (32) über eine kurze Zylinderfeder (33) mit der Schubstange (4) zum Bewegen des beweglichen Maulteils (3) gekoppelt ist, die ihrerseits zur Führung im Schieber (32) und an einem Anschlag (19) an der Basisplatte (20) geführt wird,
e) aus einer Rücklaufsperre für den Schieber (32) besteht, und hierzu an dem Schieber (32) eine Zahnstange (52) angebracht ist, in die durch einen Durchbruch durch die Basisplatte (20) hindurch die Spitze einer auf der Basisplatte (20) drehbar gelagerten Sperrklinke (50) eingreift, die bei voll zusammengezogenem Handgriff angehoben und gehalten wird, bis der Schieber (32) mit der Zahnstange (52) ganz zurückgefahren ist, worauf die Sperrklinge (50) mit ihrer Spitze erneut in die Zahnung der Zahnstange (52) eingreift,
f) aus einem Umschaltvorrichtung besteht, die aus einem zylindrischen, teilweise kreisförmigen Körper (21) und sonst mit w-förmiger Ausnehmung (28) auf der Mantelfläche und mindestens einer damit auf der Achse (25) festverbundenen Nocke (29) besteht, wobei die Umschaltvorrichtung drehbar auf der Basisplatte (20) angebracht ist, und die Arretierung oder die Freigabe der Nadel (10) in der Nadelführung (14) über die Nocke (29) und die davon angetriebene Schaltstange (5) gesteuert wird,
g) aus einer Platte (43) besteht, die parallel zur Basisplatte (20) liegt, mit einem Bolzen (42) gekoppelt ist, der auf dem Schieber (32) verankert ist und durch ein Langloch (45) in der Basisplatte (20) auf die andere Seite reicht, und durch die Achse (25) der Umschaltvorrichtung geführt wird, wozu diese Achse in einem Langloch (46) in der Platte (43) verläuft,
h) aus einem drehbar auf der Platte (43) gelagerten Schalthebel (22) besteht, an dessen freien Ende ein Schaltstift (23) angebracht ist, der durch einen Durchbruch durch die Platte (43) hindurch in die w-förmige Ausnehmung (28) eingreift, wobei der Schalthebel in seinem Lagerpunkt eine Rückstellfeder (26) aufweist, die, falls der Schaltstift frei liegt, stets auf die Längsachse des Langlochs (46) ausrichtet und rückstellt,
i) aus mindestens einer Schaltstange (5) besteht, die mit ihrem nahen Ende über eine Zylinderfeder (41) stets an die Nocke (29) gedrückt wird und mit ihrem fernen Ende die Nadel (10) einspannt oder frei gibt,
die Nadel (10) im wesentlichen nach der Kreisbahn des beweglichen Maulteils gekrümmt ist und im Bereich ihrer beiden Spitzen eine Kerbe (11) hat.

2. Chirurgisches Nähgerät nach Anspruch 1,
dadurch gekennzeichnet, daß
die Stange (4) ein verstellbares Ende (34) hat, mit dem der Öffnungswinkel des beweglichen Maulteils über die Schubweite an der Schubstange (4) eingestellt wird.

3. Chirurgisches Nähinstrument nach Anspruch 2,
dadurch gekennzeichnet, daß
die Schubstange (4) mit der Basisplatte (20) an der andern Seite des Anschlags für den Schieber (32) über eine gegenüber der Feder (33) schwächere Zylinderfeder (31) gekoppelt ist, die ein Aufklappen des mindestens einen beweglichen Maulteils unterstützt.

4. Chirurgisches Nähinstrument nach Anspruch 3,
dadurch gekennzeichnet, daß
eine Plattfeder (51) auf die Sperrklinke (50) drückt und diese in Position hält, wobei eine Nocke (56) auf dem kreisrunden Randbereich der Sperrklinke (50) zwei stabile, federnde Positionen voneinander trennt.

5. Chirurgisches Nähinstrument nach Anspruch 3,
dadurch gekennzeichnet, daß
die Feder (51) mit der Sperrklinke (50) einen bistabilen Schnappfedermechanismus bildet.

6. Chirurgisches Nähinstrument nach Anspruch 4 und 5,
dadurch gekennzeichnet, daß
die Platte (43) und die Basisplatte (20) über ein Zylinderfeder (40) gekoppelt sind, die parallel zu beiden Platten (20, 43) wirkt und diese in Richtung des geöffneten Handgriffs zurücktreibt.

7. Chirurgisches Nähinstrument nach Anspruch 6,
dadurch gekennzeichnet, daß
der Nähkopf aus einem feststehenden Maulteil (2) und einem beweglichen Maulteil (3) besteht.

8. Chirurgisches Nähinstrument nach Anspruch 7,
dadurch gekennzeichnet, daß
die Nadel (10) im feststehenden Maulteil (2) über die in die Kerbe (11) eindringende oder herausfahrende Schaltstange (5) arretiert oder freigegeben, im beweglichen Maulteil (3) über eine stellbare Feder-Stift-Anordnug (6, 7, 8) bis zu einer maximalen Klemmkraft gehalten wird, die ein zerstörungsfreies Ein- und Ausrasten der Nadel zuläßt.

9. Chirurgisches Nähinstrument nach Anspruch 6,
dadurch gekennzeichnet, daß
der Nähkopf aus zwei beweglichen Maulteilen (60, 66) besteht, in denen die Nadel über eine stellbare Feder-Stift-Anordnung (61, 62, 64) arretiert oder freigeben wird, wobei die beiden Feder-Stift-Anordnungen (61, 62, 64) über zwei gegenläufige Schaltstangen (5, 5a) betätigt werden.

10. Chirurgisches Nähinstrument nach Anspruch 9
dadurch gekennzeichnet, daß
das dickere Stiftende (64) auf der Stirnfläche (63) eines Schiebers (71, 73) gleitet, wobei der Stift (61) bei Bewegung der Maulteile (60, 66) seine Position nicht ändert.

11. Chirurgisches Nähinstrument nach Anspruch 10
dadurch gekennzeichnet, daß
die Schaltstangen (5 und 5a) am Schaftende in einem Teil (70) und (74) enden, die federnd jeweils mit dem Schieber (71 und (73) gekoppelt sind.

12. Chirurgisches Nähinstrument nach Anspruch 1
dadurch gekennzeichnet, daß
die Nadel (10) einen dreieckigen Querschnitt hat und die Kerbe (11) zylinderförmig oder prismatisch ist.

13. Chirurgisches Nähinstrument nach Anspruch 12
dadurch gekennzeichnet, daß
die Nadel (10) zu den Spitzen hin einen gratförmigen und im Bereich der Öse (13) einen flachen Rücken hat oder insgesamt einen breiten Rücken und zu den Spitzen hin einen gratförmigen Bereich hat.

14. Chirurgisches Nähinstrument nach Anspruch 13
dadurch gekennzeichnet, daß
die Nadelspitzen zum besseren Einlaufen in die Nadelführungen (14) eine ebene Anschrägung (12) haben.

## Claims

1. Surgical suturing apparatus, more especially for endoscopic surgery, including:
a) a handle with a stationary part and a displaceable part and driving and locking means mounted thereon,
b) a shaft, which is attached to the driving and locking means, and in which shaft coupling arrangements extend to a pincer-shaped sewing head mounted thereon, the sewing head lying in the extension of the shaft axis and including two jaw portions, which can be pivoted towards each other and away from each other via one of the coupling arrangements, the push-rod, at least one of the jaw portions being mounted so as to be rotatable towards and away from the shaft axis, and a needle guide at each end of the two jaw portions,
c) a needle with a central eye, which needle can be clamped in position in the needle guide in a form-locking manner with its points and can be transposed between both needle guides by operating the handle,
characterised in that
the driving and locking means, which is mounted on a base plate (20):
d) includes a rod (35), which is rigidly connected to a slide (32), for the movement of the displaceable jaw portion (3 or 60 and 66), which rod is drawn along the longitudinal axis of the base plate (20) via the handle, and the slide (32) is connected via a short cylindrical spring (33) to the push-rod (4) for moving the displaceable jaw portion (3), which push-rod, in turn, is guided on the base plate (20) for guidance in the slide (32) and on a stop member (19),
e) includes a return stop for the slide (32) and, in addition, a toothed rod (52) is mounted on the slide (32), the point of a ratchet (50) engaging in said toothed rod through an opening through the base plate (20), said ratchet being pivotably mounted on the base plate (20) and being raised and retained with a fully tightened handle until the slide (32) has returned completely with the toothed rod (52), whereupon the ratchet (50) engages again with its point in the toothing of the toothed rod (52),
f) includes a switchover device, which includes a cylindrical, partially circular body (21) and otherwise with a w-shaped recess (28) on the surface and at least one cam (29), which is securedly connected thereto on the axle (25), the switchover device being rotatably mounted on the base plate (20), and the arresting or the release of the needle (10) in the needle guide (14) being controlled via the cam (29) and the switch rod (5) driven thereby,
g) includes a plate (43), which lies parallel to the base plate (20), is connected to a pin (42), which is secured on the slide (32) and extends to the other side through an elongate slot (45) in the base plate (20), and is guided through the axle (25) of the switchover device, for which purpose this axle extends in an elongate slot (46) in the plate (43),
h) includes a shift lever (22), which is rotatably mounted on the plate (43), and on the free end of which lever a switch pin (23) is mounted, which engages in the w-shaped recess (28) through an opening through the plate (43), the shift lever having at its bearing point a return spring (26) which, if the switch pin is exposed, always aligns with, and returns to, the longitudinal axis of the elongate slot (46), and
i) includes at least one switch rod (5), which is always pressed against the cam (29) with its near end via a cylindrical spring (41) and clamps in position or releases the needle (10) with its far end,
the needle (10) being curved substantially according to the circular path of the displaceable jaw portion and having a notch (11) in the region of its two points.

2. Surgical suturing apparatus according to claim 1, characterised in that the rod (4) has a displaceable end (34), by means of which the angle of opening of the displaceable jaw portion is adjusted over the slide width at the push-rod (4).

3. Surgical suturing instrument according to claim 2, characterised in that the push-rod (4) is connected to the base plate (20) on the other side of the stop member for the slide (32) via a cylindrical spring (31), which is weaker than the spring (33) and assists an opening of at least one displaceable jaw portion.

4. Surgical suturing instrument according to claim 3, characterised in that a flat spring (51) presses upon the ratchet (50) and retains the latter in position, a cam (56) on the circular edge region of the ratchet (50) separating two stable, resilient positions from each other.

5. Surgical suturing instrument according to claim 3, characterised in that the spring (51) forms with the ratchet (50) a bistable catch-spring mechanism.

6. Surgical suturing instrument according to claims 4 and 5, characterised in that the plate (43) and the base plate (20) are connected via a cylindrical spring (40), which acts parallel to both plates (20, 43) and urges said plates back in the direction towards the opened handle.

7. Surgical suturing instrument according to claim 6, characterised in that the sewing head includes a stationary jaw portion (2) and a displaceable jaw portion (3).

8. Surgical suturing instrument according to claim 7, characterised in that the needle (10) is arrested or released in the stationary jaw portion (2) via the switch rod (5), which penetrates the notch (11) or emerges therefrom, is retained in the displaceable jaw portion (3) via an adjustable spring-and-pin arrangement (6, 7, 8) up to a maximum clamping force which permits a non-destructive engagement and disengagement of the needle.

9. Surgical suturing instrument according to claim 6, characterised in that the sewing head includes two displaceable jaw portions (60, 66), in which the needle is arrested or released via an adjustable spring-and-pin arrangement (61, 62, 64), the two spring-and-pin arrangements (61, 62, 64) being actuated via two oppositely directed switch rods (5, 5a).

10. Surgical suturing instrument according to claim 9, characterised in that the thicker pin end (64) slides along the end face (63) of a slide (71, 73), the pin (61) not changing its position when the jaw portions (60, 66) move.

11. Surgical suturing instrument according to claim 10, characterised in that the switch rods (5 and 5a) terminate at the shaft end in a portion (70) and (74), which portions are each resiliently connected to the slide (71) and (73).

12. Surgical suturing instrument according to claim 1, characterised in that the needle (10) has a triangular cross-section, and the notch (11) is cylindrical or prismatic.

13. Surgical suturing instrument according to claim 12, characterised in that the needle (10) has a fin-shaped ridge towards the points and a flat ridge in the region of the eye (13) or has a wide ridge generally and a fin-shaped region towards the points.

14. Surgical suturing instrument according to claim 13, characterised in that the needle points have a flat bevel (12) for better insertion into the needle guides (14).

## Revendications

1. Instrument chirurgical de suture, en particulier pour la chirurgie endoscopique, se composant d'
a) une poignée avec une pièce fixe et une pièce mobile et d'un mécanisme de mouvement et de verrouillage,
b) une tige appliquée au mécanisme de mouvement et de verrouillage, dans laquelle des dispositifs de couplage se développent en une tête de suture rapportée, en forme de pince, la tête de suture située dans le prolongement de l'axe de la tige, se composant de deux parties de mâchoire l'une au-dessus de l'autre pouvant s'ouvrir et se fermer, par l'intermédiaire d'un des dispositifs de couplage de la barre de poussée dont l'une au moins est montée sur l'axe de la tige en pouvant se rapprocher et s'éloigner par rotation et d'un guide-aiguille pour chacune d'elle à l'extrémité des deux pièces de mâchoire,
c) une aiguille avec un oeillet central qui est serré par ses pointes dans le guide-aiguille par une liaison de forme et entre les deux guidages d'aiguille peut être déplacée par actionnement de la poignée,
caractérisé en ce que
- le mécanisme de commande et de verrouillage qui est monté sur une plaque de base (20)
d) se compose d'une barre (35) rigide accouplée à un poussoir (32) pour le mouvement de la pièce de mâchoire mobile (3 ou 60 ou 66), qui est tirée par la poignée le long de l'axe longitudinal de la plaque de base (20) et le poussoir (32) est accouplé par un court ressort cylindrique (33) à la barre de poussée (4) pour le mouvement de la pièce mobile de mâchoire (3), qui de son côté pour la conduite du poussoir (32) est guidée contre la butée (19) de la plaque de base (20),
e) se compose d'un cliquet antiretour pour le poussoir (32) et pour cela est appliquée que le poussoir (32) une crémaillère (52), dans laquelle est engagée à travers une ouverture dans la plaque de base (20) la pointe d'un cliquet de blocage (50) monté mobile en rotation sur la plaque de base (20), qui est soulevé et maintenu lorsque la poignée est complètement contractée, jusqu'à ce que le poussoir (32) avec la crémaillère soit complètement reculé, après quoi le cliquet d'arrêt (50) avec sa pointe s'encliquette à nouveau dans la denture de la crémaillère,
f) se compose d'un dispositif de commutation, qui comprend un corps (21) cylindrique, partiellement circulaire et d'un évidement (28) habituellement en forme de (w) (28) sur la surface enveloppe et au moins d'une came (29) reliée fixement à l'axe (25), le dispositif de commutation étant monté mobile en rotation sur la plaque de base (20), et le blocage ou le déverrouillage de l'aiguille (10) dans le guide-aiguille (14) étant commandé par la came (29) et la barre de couplage (5) mise en mouvement par celle-ci,
g) se compose d'une plaque (43), située parallèlement à la plaque de base (20) accouplée à un axe (42), qui est ancré sur le poussoir (32) et à travers un trou oblong (45) atteint dans la plaque de base (20) son autre face et est guidée par l'axe (25) du dispositif de communtation, vu que cet axe se développe dans un trou oblong (46) de la plaque (43),
h) se compose d'un levier (22) monté mobile en rotation sur la plaque (43), à l'extrémité libre duquel est appliquée une cheville de manoeuvre (23), qui s'encliquette à travers une ouverture de la plaque (43) dans l'évidement (28) en forme de (w), le levier de commutation comportant dans son lieu de fixation un ressort de rappel (26), qui, si la cheville de manoeuvre est libre, l'ajuste toujours et la rappelle sur l'axe longitudinal du trou oblong (46),
i) se compose d'au moins une barre de couplage (5) qui est toujours comprimée avec son extrémité libre par un ressort cylindrique (41) contre la came (29) et par son extrémité éloignée bloque l'aiguille (10) ou la libère,
- l'aiguille (10) est courbée essentiellement selon l'orbite de la pièce de mâchoire mobile et a une encoche (11) dans la zone de ses deux pointes.

2. Instrument chirurgical de suture selon la revendication 1,
caractérisé en ce que
la barre (4) a une extrémité réglable (34), avec laquelle on ajuste l'angle d'ouverture de la pièce mobile de mâchoire par l'étendue de poussée sur la barre de poussée (4).

3. Instrument chirurgical de suture selon la revendication 2,
caractérisé en ce que
la base de poussée (4) est accouplée à la plaque de base (20) de l'autre côté de la butée pour le poussoir (32) par l'intermédiaire d'un ressort cylindrique (31) plus faible que le ressort (33), qui appuie une ouverture d'au moins une pièce de mâchoire mobile.

4. Instrument chirurgical de mesure selon la revendication 3,
caractérisé en ce qu'
un ressort plat (51) comprime le cliquet d'arrêt (50) et maintient celui-ci en position, une came (56) séparant l'une de l'autre sur la zone de bord du cliquet d'arrêt (50) deux positions élastiques, stables.

5. Instrument de suture chirurgical selon la revendication 3,
caractérisé en ce que
le ressort (51) forme avec le cliquet d'arrêt (50) un mécanisme bistable de ressort à déclic.

6. Instrument de suture chirurgical selon les revendications 4 et 5,
caractérisé en ce que
la plaque (43) et la plaque de base (20) sont accouplées par un ressort cylindrique (40), qui agit parallèlement aux deux plaques (20, 43) et fait reculer celles-ci en direction de la poignée ouverte.

7. Instrument de suture chirurgical selon la revendication 6,
caractérisé en ce que
la tête de suture se compose d'une pièce de mâchoire fixe (2) et d'une pièce de mâchoire mobile (3).

8. Instrument de suture chirurgical selon la revendication 7,
caractérisé en ce que
l'aiguille (10) dans la pièce de mâchoire fixe (2) est bloquée ou libérée par la barre de couplage (5) pénétrant ou sortant de l'encoche (11), dans la pièce de mâchoire mobile (3), elle est maintenue par un dispositif ressort-cheville réglable (6, 7, 8) jusqu'à une force de serrage maximale qui admet un verrouillage et un déverrouillage de l'aiguille sans destruction.

9. Instrument de suture chirurgical selon la revendication 6,
caractérisé en ce que
la tête de suture se compose de deux parties de mâchoire (60, 66) mobiles, dans lesquelles l'aiguille est arrêtée ou libérée par un dispositif cheville-ressort réglable (61, 62, 64), les deux dispositifs ressort-cheville (61, 62, 64) étant actionnés par deux barres de couplage en sens opposés.

10. Instrument de suture chirurgical selon la revendication 9,
caractérisé en ce que
l'extrémité épaisse de la cheville (64) glisse sur la face frontale (63) d'un poussoir (71, 73), la cheville (61) ne changeant pas de position lors du mouvement des pièces de mâchoire (60, 66).

11. Instrument chirurgical de suture selon la revendication 10,
caractérisé en ce que
les barres de couplage (5 et 5a) se terminent à l'extrémité de tige dans une partie (70) et (74), qui sont accouplées de manière élastique respectivement avec le poussoir (71) et (73).

12. Instrument de suture chirurgical selon la revendication 1,
caractérisé en ce que
l'aiguille (10) a une section transversale triangulaire et l'encoche (11) est cylindrique ou prismatique.

13. Instrument de suture chirurgical selon la revendication 12,
caractérisé en ce que
l'aiguille (10) a en direction des pointes une forme d'arête et dans la zone de l'oeillet (13) un dos plat ou au total un dos large et en direction des pointes une zone en forme d'arête.

14. Instrument de suture chirurgical selon la revendication 13,
caractérisé en ce que
les pointes de l'aiguille ont une partie oblique plane (12) pour leur meilleure introduction dans le guide-aiguille (14).
